(19) Europäisches Patentamt
European Patent Office
Office européen des brevets

(11) Veröffentlichungsnummer: **0 108 352**
**B1**

# (12) EUROPÄISCHE PATENTSCHRIFT

(45) Veröffentlichungstag der Patentschrift: **02.04.86**

(51) Int. Cl.⁴: **C 07 D 253/08**, A 61 K 31/53

(21) Anmeldenummer: **83110779.2**

(22) Anmeldetag: **28.10.83**

## (54) 3-(3-Jodpropargyl)-benzo-1,2,3-triazin-4-one, Verfahren zu ihrer Herstellung und ihre Verwendung in mikrobiziden Mitteln.

(30) Priorität: **09.11.82 DE 3241265**

(43) Veröffentlichungstag der Anmeldung: **16.05.84 Patentblatt 84/20**

(45) Bekanntmachung des Hinweises auf die Patenterteilung: **02.04.86 Patentblatt 86/14**

(84) Benannte Vertragsstaaten: **BE CH DE FR GB IT LI NL SE**

(56) Entgegenhaltungen:
**DE - A - 2 209 470**
**DE - A - 2 910 220**
**US - A - 3 316 262**
**US - A - 3 446 797**

**CHEMICAL ABSTRACTS, Band 97, Nr. 3, 19. Juli 1982, Seite 713, Nr. 23779c, Columbus, Ohio, US**

(73) Patentinhaber: **BAYER AG, Konzernverwaltung RP Patentabteilung, D-5090 Leverkusen 1 Bayerwerk (DE)**

(72) Erfinder: **Schmitt, Hans-Georg, Dr., Gustav-Freytag-Strasse 2, D-5090 Leverkusen 1 (DE)**
Erfinder: **Paulus, Wilfried, Dr., Deswatinesstrasse 90, D-4150 Krefeld 1 (DE)**
Erfinder: **Genth, Hermann, Dr., Am Heckerhof 60, D-4150 Krefeld 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung betrifft neue 3-(3-Jodpropargyl)-benzo-1,2,3-triazin-4-one, Verfahren zu ihrer Herstellung und ihre Verwendung in mikrobiziden Mitteln.

Aus CA *97*, S. 713, Nr. 23779c (1982) sind 6-substituierte 3-(3-Jodpropargyl)-2-(3H)-benzothiazole bekannt. Aus der US-PS 3.446.797 sind ebenfalls spezielle die Jodpropargylgruppe enthaltende Heterocyclen bekannt.

Die neuen 3-(3-Jodpropargyl)-benzo-1,2,3-triazin-4-one entsprechen der Formel:

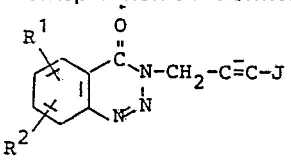

in der

$R^1$ Wasserstoff, $C_1$-$C_6$-Niederalkyl, Halogen, Cyano, Nitro, $C_1$-$C_6$-Niederalkoxy, $C_6$-$C_{18}$-Aryloxy, $C_1$-$C_6$-Niederalkylthio, $C_6$-$C_{18}$-Arylthio, Chlorphenylthio, $C_1$-$C_{10}$-Alkoxycarbonyl, $C_1$-$C_6$-Niederalkylsulfonyl, $C_6$-$C_{18}$-Arylsulfonyl oder Chlorphenylsulfonyl bedeutet, und

$R^2$ Wasserstoff, $C_1$-$C_6$-Niederalkyl oder Halogen bedeutet.

Niederalkyl kann hierbei erfindungsgemäss ein geradkettiger oder verzweigter Kohlenwasserstoffrest mit 1 bis 6 Kohlenstoffatomen sein. Beispielsweise seien die folgenden Niederalklyreste genannt: Methyl, Ethyl, Propyl, Isopropyl, Butyl, Isobutyl, Pentyl, Isopentyl, Hexyl und Isohexyl. Bevorzugte Niederalkylreste sind der Methyl- und der Ethylrest.

Niederalkoxy kann hierbei erfindungsgemäss ein über Sauerstoff gebundener, geradkettiger oder verzweigter Kohlenwasserstoffrest mit 1 bis 6 Kohlenstoffatomen sein. Beispielsweise seien die folgenden Niederalkoxyreste genannt: Methoxy, Ethoxy, Ethoxy, Propoxy, Isopropoxy, Butoxy, Isobutoxy, Pentoxy, Isopentoxy, Hexoxy und Isohexoxy. Bevorzugt sind die Methoxy- und die Ethoxygruppe.

Aryloxy kann hierbei erfindungsgemäss ein über Sauerstoff gebundener, aromatischer Rest aus der Benzolreihe mit 6 bis 18 Kohlenstoffatomen sein. Beispielsweise seien die folgenden Aryloxyreste genannt: Phenoxy und Naphthoxy.

Bevorzugt wird der Phenoxyrest.

Niederalkylthio kann hierbei erfindungsgemäss ein über Schwefel gebundener, geradkettiger oder verzweigter Kohlenwasserstoffrest mit 1 bis 6 Kohlenstoffatomen sein. Beispielsweise seien die folgenden Niederalkylthioreste genannt: Methylthio, Ethylthio, Propylthio, Isopropylthio, Butylthio, Isobutylthio, Pentylthio, Isopentylthio, Hexylthio und Isohexylthio. Bevorzugt sind der Methylthio und der Ethylthiorest.

Arylthio kann hierbei erfindungsgemäss ein über Schwefel gebundener, aromatischer Rest aus der Benzolreihe mit 6 bis 18 Kohlenstoffatomen sein. Beispielsweise seien die folgenden Arylthioreste genannt: Phenylthio, Naphthylthio und Tolylthio.

Bevorzugt ist der Phenylthiorest.

Alkoxycarbonyl kann hierbei erfindungsgemäss ein Rest der Formel:

$$R^5-O-\overset{\overset{O}{\|}}{C}-$$

in der $R^5$ ein geradkettiger oder verzweigter Kohlenwasserstoffrest mit 1 bis 10, bevorzugt 1 bis 6 Kohlenstoffatomen ist.

Beispielsweise seien die folgenden Alkoxycarbonylreste genannt: Methoxycarbonyl, Ethoxycarbonyl, Propoxycarbonyl, Isopropoxycarbonyl, Butoxycarbonyl, Isobutoxycarbonyl, Pentoxycarbonyl, Hexoxycarbonyl.

Bevorzugtes Alkoxycarbonyl ist Methoxycarbonyl.

Niederalkylsulfonyl kann hierbei erfindungsgemäss ein durch einen geradkettigen oder verzweigten Kohlenwasserstoffrest mit 1 bis 6 Kohlenstoffatomen substituierter Sulfonylrest sein. Beispielsweise seien die folgenden Niederalkylsulfonylreste genannt: Methylsulfonyl, Ethylsulfonyl, Propylsulfonyl, Butylsulfonyl.

Bevorzugtes Alkylsulfonyl ist Methylsulfonyl.

Arylsulfonyl kann hierbei erfindungsgemäss ein durch einen aromatischen Rest aus der Benzolreihe mit 6 bis 18 Kohlenstoffatomen substituierter Sulfonylrest sein. Beispielsweise seien die folgenden Reste genannt: Phenylsulfonyl, Naphthylsulfonyl und Tolylsulfonyl.

Bevorzugtes Arylsulfonyl ist Phenylsulfonyl.

Erfindungsgemäss werden 3-(3-Jodpropargyl)-benzo-1,2,3-triazin-4-one der Formel:

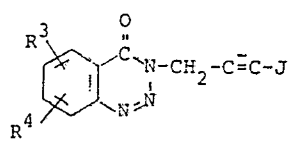

in der

$R^3$ Wasserstoff, $C_1$-$C_6$-Niederalkyl, Fluor, Chlor, Brom oder Nitro bedeutet, und $R^4$ Wasserstoff, $C_1$-$C_6$-Niederalkyl oder Chlor bedeutet, bevorzugt.

Beispielsweise seien die folgenden 3-(3-Jodpropargyl)-benzo-1,2,3-triazin-4-one genannt:

3-(3-Jodpropargyl)-benzo-1,2,3-triazin-4-on

5-Chlor-3-(3-jodpropargyl)-benzo-1,2,3-triazin-4-on

6-Chlor-3-(3-jodpropargyl)-benzo-1,2,3-triazin-4-on

7-Chlor-3-(3-jodpropargyl)-benzo-1,2,3-triazin-4-on

7-Fluor-3-(3-jodpropargyl)-benzo-1,2,3-triazin-4-on

7-Brom-3-(3-jodpropargyl)-benzo-1,2,3-triazin-4-on

6,8-Dichlor-3-(3-jodpropargyl)-benzo-1,2,3-triazin-4-on

6,8-Dibrom-3-(3-jodpropargyl)-benzo-1,2,3-triazin-4-on

3-(3-Jodpropargyl)-6-nitro-benzo-1,2,3-triazin-4-on

3-(3-Jodpropargyl)-5-methyl-benzo-1,2,3-triazin-4-on

Die neuen 3-(3-Jodpropargyl)-benzo-1,2,3-triazin-4-one können hergestellt werden, indem man Benzo-1,2,3-triazin-4-one der Formel:

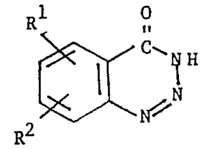

in der $R^1$ und $R^2$ die obengenannte Bedeutung haben,
mit einer Proparglyverbindung der Formel:

$$X^1-CH_2-C\equiv C-J$$

in der $X^1$ für Halogen, Alkylsulfonyloxy oder Arylsulfonyloxy steht,
in Gegenwart einer Base umsetzt.

Das erfindungsgemässe Verfahren kann anhand des folgenden Formelschemas erläutert werden:

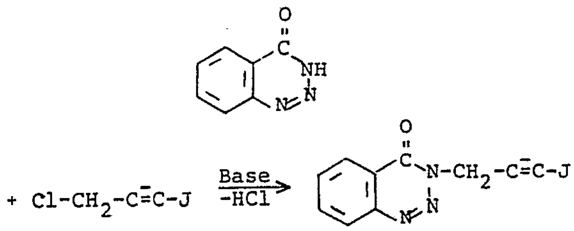


In einer anderen Ausführungsform des erfindungsgemässen Verfahrens stetzt man Salze der Benzo-1,2,3-triazin-4-one der Formel:

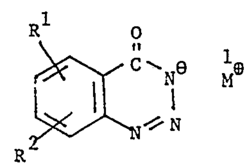

in der
$R^1$ und $R^2$ die obengenannte Bedeutung haben, und
$M^{1\oplus}$ für ein Metallion steht,
mit einer Propargylverbindung der Formel:

$$X^1-CH_2-C\equiv C-J$$

in der $X^1$ die obengenannte Bedeutung hat, um.

Diese Ausführungsform des erfindungsgemässen Verfahrens kann durch die folgende Gleichung erläutert werden:

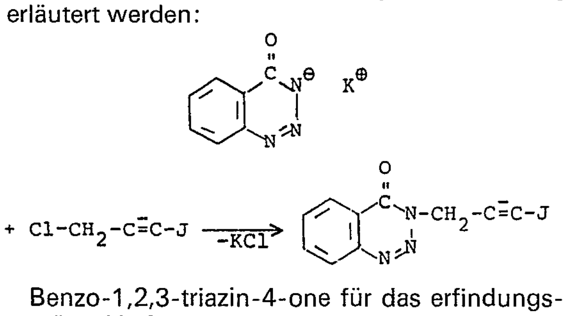


Benzo-1,2,3-triazin-4-one für das erfindungsgemässe Verfahren sind an sich bekannt (z. B. „J. Org. Chem.", *26*, 619 [1961]). Sie können beispielsweise hergestellt werden, indem man ausgehend von den entsprechenden Anthranilsäuren mit Phosgen in Gegenwart eines säurebindenden Mittels die Isatosäureanhydride herstellt, die mit Ammoniak zu Anthranilamiden gespalten werden. Diese können durch Diazotierung mit Natriumnitrit in wässeriger Salzsäurelösung in die Benzo-1,2,3-triazin-4-one übergeführt werden.

Als Salze der Benzo-1,2,3-triazin-4-one werden im allgemeinen die Alkalisalze, im besonderen die Natrium- und Kaliumsalze, bevorzugt. Diese Salze lassen sich durch Hinzufügen einer entsprechenden Base, z. B. Natriumhydroxid oder Kaliumhydroxid, in wässeriger, alkoholischer oder etherischer Verdünnung aus den Benzo-1,2,3-triazin-4-onen herstellen.

Für das erfindungsgemässe Verfahren werden Benzo-1,2,3-triazin-4-one der Formel:

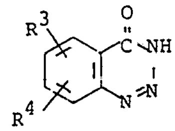

in der $R^3$ und $R^4$ die obengenannte Bedeutung haben,
und Salze der Benzo-1,2,3-triazin-4-one der Formel:

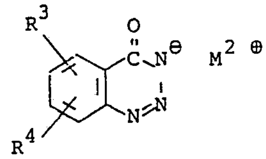

in der
$R^3$ und $R^4$ die obengenannte Bedeutung haben, und
$M^{2\oplus}$ für ein Alkali- oder ein Erdalkaliion steht,
bevorzugt.

Beispielsweise seien die folgenden Benzo-1,2,3-triazin-4-one genannt:
Benzo-1,2,3-triazin-4-on, 5-Chlor-benzo-1,2,3-triazin-4-on, 6-Chlor-benzo-1,2,3-triazin-4-on, 7-Chlor-benzo-1,2,3-triazin-4-on, 7-Fluor-benzo-1,2,3-triazin-4-on, 7-Brom-benzo-1,2,3-triazin-4-on, 6-Nitro-benzo-1,2,3-triazin-4-on, 6,8-Dichlor-benzo-1,2,3-triazin-4-on, 6,8-Dibrom-benzo-1,2,3-triazin-4-on, 5-Methyl-benzo-1,2,3-triazin-4-on.

Als Salze der Benzo-1,2,3-triazin-4-one seien beispielsweise die Natrium- und Kaliumsalze der genannten Verbindungen genannt.

Jodpropargylverbindungen des erfindungsgemässen Verfahrens sind ebenfalls an sich bekannt und können beispielsweise durch Umsetzung von 3-Chlor-propin mit Jod in wässerig-alkalischer Lösung bei 0 bis 5° C hergestellt werden („J. Am. Chem. Soc.", *77*, 176 [1955]) oder durch Umsetzung von 3-Jodpropargylalkohol mit Benzolsulfonylchlorid erhalten werden (DE-OS 2910220).

Beispielsweise seien die folgenden Jodpropargylverbindungen genannt: 3-Chlor-1-jodpropin, 3-Brom-1-jodpropin, 1,3-Dijodpropin, Benzolsulfonsäure-3-jodpropargylester, 4-Toluolsulfonsäure-3-jodpropargylester und Methansulfonsäure-3-jodpropargylester.

Für das erfindungsgemässe Verfahren werden Jodpropargylverbindungen der Formel:

$$X^2-CH_2-C\equiv C-J$$

in der $X^2$ für Chlor, Brom und Phenylsulfonyloxy steht,
bevorzugt.

Im besonderen bevorzugt werden 3-Chlor-1-jodpropin und 3-Brom-1-jodpropin.

Die Umsetzung von Benzo-1,2,3-triazin-4-onen mit Jodpropargylverbindungen wird in Gegenwart von Basen durchgeführt, die den entstehenden Halogenwasserstoff bzw. die Sulfonsäure binden. Als Basen seien Alkalicarbonate und Erdalkalicarbonate sowie tertiäre Amine genannt. Alkalicarbonate sind hierbei im wesentlichen Natrium- und Kaliumcarbonat. Erdalkalicarbonate sind hierbei im wesentlichen Magnesium- und Calciumcarbonat. Tertiäre Amine sind hierbei im wesentlichen durch geradkettige oder verzweigte Niederalkylreste (1 bis etwa 6 Kohlenstoffatome) substituierte Amine wie Triethylamin.

Das erfindungsgemässe Verfahren wird im allgemeinen im Temperaturbereich von 20 bis 100, vorzugsweise von 40 bis 80° C, durchgeführt.

Im allgemeinen wird das erfindungsgemässe Verfahren bei Normaldruck durchgeführt. Es ist aber auch möglich, das erfindungsgemässe Verfahren bei einem Unter- bzw. bei einem Überdruck, beispielsweise im Druckbereich von 0,5 bis 5 bar, durchzuführen.

Für das erfindungsgemässe Verfahren werden im allgemeinen das Benzo-1,2,3-triazin-4-on bzw. dessen Salz und die Jodpropargylverbindung in etwa äquimolaren Mengen eingesetzt. Es ist jedoch auch möglich, die eine oder die andere Komponente im Überschuss einzusetzen.

Das erfindungsgemässe Verfahren kann in Gegenwart eines Lösungs- oder Verdünnungsmittels durchgeführt werden. Lösungs- oder Verdünnungsmittel für das erfindungsgemässe Verfahren sind Verbindungen, die sich unter den erfindungsgemässen Bedingungen nicht verändern. Beispielsweise seien Alkohole wie Ethanol, Propanol und Butanol, Ether, wie Dioxan und Tetrahydrofuran, Kohlenwasserstoffe wie Toluol, Chlorkohlenwasserstoffe wie Chloroform, Ketone wie Aceton, oder Amide wie Dimethylformamid, genannt.

Das erfindungsgemässe Verfahren kann beispielsweise wie folgt ausgeführt werden:

Das Benzo-1,2,3-triazin-4-on wird ggf. in einem Lösungsmittel gelöst bzw. suspendiert und die Base zugegeben. Dann tropft man die Jodpropargylverbindung bei der Reaktionstemperatur zu. Nach Beendigung der Umsetzung wird das Reaktionsgemisch in Wasser eingetragen. Die 3-(3-Jodpropargyl)-benzo-1,2,3-triazin-4-on-Verbindung fällt aus und kann in üblicher Weise abgetrennt und gereinigt werden.

Die erfindungsgemässen 3-(3-Jodpropargyl)-benzo-1,2,3-triazin-4-one können als Wirkstoffe zur Bekämpfung von Mikroorganismen, im besonderen in technischen Materialien, verwendet werden.

Technische Materialien sind nicht lebende Materialien, die für die Verwendung in der Technik zubereitet worden sind. Beispielsweise können technische Materialien, die durch den erfindungsgemässen Wirkstoff vor einer mikrobiellen Veränderung und Zerstörung geschützt werden sollen, Klebstoffe, Leime, Papiere und Kartone, Textilien, Leder, Holz, Anstrichmittel, Baustoffe, Kautschuk und Kunststoffartikel, Kühlschmiermittel und andere Materialien sein, die durch Mikroorganismen zersetzt werden können. Im Rahmen der zu schützenden Materialien seien auch Teile von Produktionsanlagen, beispielsweise Kühlwasserkreisläufe, genannt, die durch Mikroorganismen beeinträchtigt sein können. Im Rahmen der vorliegenden Erfindung seien als technische Materialien vorzugsweise aus Holz gefertigte Artikel sowie Anstrich- und Beschichtungsmittel genannt.

Mikroorganismen, die einen Abbau oder eine Veränderung der technischen Materialien bewirken können, sind beispielsweise Pilze, Bakterien, Hefen, Algen und Schleimorganismen. Vorzugsweise wirken die erfindungsgemässen Substanzen gegen Pilze.

Es seien beispielsweise Mikroorganismen der folgenden Gattungen genannt:

*Alternaria*, wie *Alternaria tenuis*,
*Aspergillus*, wie *Aspergillus niger*,
*Chaetomium*, wie *Chaetomium globosum*,
*Coniophora*, wie *Coniophora cerebella*,
*Lentinus*, wie *Lentinus tigrinus*,
*Penicillium*, wie *Penicillium glaucum*,
*Polyporus*, wie *Polyporus versicolor*,
*Aureobasidium*, wie *Aureobasidium pullulans*,
*Sclerophoma*, wie *Sclerophoma pityophila*.

Je nach ihrem Anwendungsgebiet können die erfindungsgemässen Wirkstoffe in die üblichen Formulierungen überführt werden, wie Lösungen, Emulsionen, Suspensionen, Pulver, Pasten und Granulate. Diese können in an sich bekannter Weise hergestellt werden, z. B. durch Vermischen der Wirkstoffe mit einem Streckmittel, aus flüssigem Lösungsmittel und/oder festen Trägerstoffen, ggf. unter Verwendung von oberflächenaktiven Mitteln, wie Emulgatoren und/oder Dispergiermitteln, wobei beispielsweise im Falle der Benutzung von Wasserstreckmitteln ggf. organische Lösungsmittel als Hilfsmittel verwendet werden können.

Feste Trägerstoffe, die bei der Herstellung der fertigen Anwendungsform des Wirkstoffes zugegeben werden, können beispielsweise feinteilige Aluminiumoxide, Silikate, Carbonate, Eisenoxide, Gips oder Holzmehl sein.

Oberflächenaktive Mittel können handelsübliche Emulgatoren, wie Aryl- oder Alkylsulfonate, ethoxylierte Alkylphenole, Fettalkohole oder Alkylamine oder Dispergiermittel, wie Polycarbonsäureester, Polyvinylalkohol, Lignin, Sulfitablaugen oder Methylcellulose sein.

Organische Lösungsmittel für die Wirkstoffe können beispielsweise Alkohole, wie niedere aliphatische Alkohole, vorzugsweise Ethanol und Isopropanol, oder Benzylalkohol, Ketone wie Aceton oder Methylethylketon, flüssige Kohlenwasserstoffe, wie Benzinfraktionen, und chlorierte Kohlenwasserstoffe, wie 1,2-Dichlorethan, sein.

Die Anwendungsformen des erfindungsgemässen mikrobiziden Mittels enthalten im allgemeinen 5 bis 100 Gew.-%, bevorzugt 20 bis 90 Gew.-%, des 3-(3-Jodpropargyl)-benzo-1,2,3-triazin-4-

ons als Wirkstoff. Die für den Schutz technischer Materialien erforderliche Anwendungskonzentration der erfindungsgemässen Wirkstoffe richtet sich nach der Art und dem Vorkommen der zu bekämpfenden Mikroorganismen sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5, vorzugsweise von 0,05 bis 1 Gew.-%, bezogen auf das zu schützende Material.

Die erfindungsgemässen Wirkstoffe können in den Formulierungen in Mischungen mit anderen bekannten anorganischen oder organischen Fungiziden und/oder Insektiziden vorliegen. Beispielsweise seien die folgenden Wirkstoffe genannt: Phenolderivate, Formaldehyd-abspaltende Verbindungen, Dithiocarbonate, Benzimidazolylcarbamate, Thiazolylbenzimidazol, Isothiazolon- und Benzisothiazolonderivate, Trihalogenmethylthioverbindungen, Tetrachlorisophthalsäuredinitril, Mercaptobenzthiazol und Mercaptopyridin.

Der erfindungsgemässe 3-(3-Jodpropargyl)-benzo-1,2,3-triazin-4-on-Wirkstoff zeichnet sich durch eine hohe Wirksamkeit im Vergleich zu ähnlichen Wirkstoffen aus. Im besonderen zeigt er eine hohe Stabilität in alkalischen Medien, wodurch die Verwendbarkeit als Mikrobizid zum Schutz technischer Materialien wesentlich verbreitert wird.

*Herstellung*

*Beispiel 1:*

8,38 g (0,038 Mol) 7-Chlor-benzo-1,2,3-triazin-4-on-Kaliumsalz werden in 150 ml DMF suspendiert. Bei 20° C werden 8 g (0,0399 Mol) 3-Chlor-1-jodpropin zugetropft. Es wird etwa 8 Stunden weitergerührt, dann die Reaktionsmischung in 600 ml Wasser eingetragen; die ausgefallenen Kristalle werden abgesaugt und an der Luft getrocknet. Zur Reinigung wird das Produkt in Dioxan gelöst, kurz unter Zusatz von etwas Aktivkohle gerührt und nach Filtration die Lösung eingeengt. Man erhält 7 g (53,3% der Theorie) 7-Chlor-3-(3-jodpropargyl)-benzo-1,2,3-triazin-4-on, schwach gelbe Kristalle vom Schmp. 157-159° C.

*Beispiele 2 bis 5:*

In gleicher Weise wie in Beispiel 1 werden die folgenden Verbindungen hergestellt:

2) 3-(3-Jodpropargyl)-benzo-1,2,3-triazin-4-on; Schmp. 142-144° C

3) 3-(3-Jodpropargyl)-6-nitro-benzo-1,2,3-triazin-4-on; Schmp. 178-180° C

4) 6-Chlor-3-(3-jodpropargyl)-benzo-1,2,3-triazin-4-on; Schmp. 172-175° C

5) 6,8-Dichlor-3-(3-jodpropargyl)-benzo-1,2,3-triazin-4-on; Schmp. 172-174° C (Aus Aceton)

*Anwendungsbeispiele*

Es werden die folgenden Wirkstoffe angewandt:

A) 3-(3-Jodpropargyl)-benzo-1,2,3-triazin-4-on

B) 3-(3-Jodpropargyl)-6-nitro-benzo-1,2,3-triazin-4-on

C) 3-(3-Jodpropargyl)-7-Chlor-benzo-1,2,3-triazin-4-on

D) Zum Vergleich: N-Butyl-jodpropargyl-carbamat (Polyphase Antimildew der Fa. Troy Chem. Corp. USA)

*Beispiel 6:*

Zum Nachweis der Wirksamkeit gegen Pilze werden die minimalen Hemmkonzentrationen (MHK) von erfindungsgemässen Wirkstoffen bestimmt:

Ein Agar, der aus Bierwürze und Pepton hergestellt wird, wird mit erfindungsgemässen Wirkstoffen in Konzentrationen von 0,1 bis 5000 mg/l versetzt. Nach Erstarren des Agars erfolgt Kontamination mit Reinkulturen der in der Tabelle aufgeführten Testorganismen. Nach 2wöchiger Lagerung bei 28° C und 60 bis 70% rel. Luftfeuchtigkeit wird die MHK bestimmt. MHK ist die niedrigste Konzentration an Wirkstoff, bei der keinerlei Bewuchs durch die verwendete Mikrobenart erfolgt; MHK-Werte sind in den nachstehenden Tabellen zusammengefasst.

*Tabelle I*

MHK-Wert (mg/l)

| Testorganismen | Wirkstoff | | | |
|---|---|---|---|---|
| | A | B | C | D (Vergleich) |
| *Alternaria tenuis* | 5 | | | |
| *Aspergillus niger* | 5 | <20 | <20 | 80 |
| *Aureobasidium pullulans* | 5 | | | |
| *Chaetomium globosum* | <20 | <20 | <20 | 60 |
| *Coniophora cerebella* | 0,5 | | | |
| *Lentinus tigrinus* | 2 | | | |
| *Penicillium glaucum* | 5 | <20 | <20 | 40 |
| *Sclerophoma pityophila* | 3,5 | | | |
| *Trichoderma viride* | 10 | | | 140 |

*Beispiel 7:*

(Wirkung gegen Schleimorganismen)

Erfindungsgemässe Verbindungen, in wenig Aceton gelöst, werden in Konzentrationen von jeweils 0,1 bis 100 mg/l in Allens Nährlösung („Arch. Mikrobiol.", *17*, 34 bis 53 [1952]), die in 4 l sterilem Wasser, 0,2 g Ammoniumchlorid, 4,0 g Natriumnitrat, 1,0 g Dikaliumhydrogenphosphat, 0,2 g Calciumchlorid, 2,05 g Magnesiumsulfat, 0,02 g Eisenchlorid und 1% Caprolactam enthält, zur Anwendung gebracht.

Kurz vorher wird die Nährlösung mit Schleimor-

ganismen (ca. $10^6$ Keime/ml) infiziert, die aus bei der Polyamidherstellung verwendeten Spinnwasser-Kreisläufen isoliert wurden. Nährlösungen, die die minimale Hemmkonzentration (MHK) oder grössere Wirkstoffkonzentrationen aufweisen, sind nach 3wöchiger Kultur bei Raumtemperatur noch völlig klar, d. h. die in wirkstofffreien Nährlösungen nach 3 bis 4 Tagen bemerkbare starke Vermehrung der Mikroben und Schleimbildung unterbleibt.

*Tabelle II*

MHK-Wert (mg/l)

| Wirkstoff | MHK (mg/l) |
|---|---|
| A | 5 |
| B | 20 |
| C | 30 |

*Beispiel 8:*

Prüfung von Anstrichen auf Schimmelfestigkeit

Die Prüfung wird in Anlehnung an den Report 219 der Defense Standards Laboratories Maibyrnong/Australien wie folgt durchgeführt: Ein glatter Karton wird beidseitig mit dem zu prüfenden Produkt bestrichen und 8 Tage bei Raumtemperatur getrocknet. Zur Alterung wird ein Teil des Anstriches 24 Stunden in fliessendem Wasser von 24° C gewässert oder 8 Tage mit Frischluft von 40 bis 60° C belüftet oder 110 Stunden einem trockenen Xenon-Test ausgesetzt. Von den so vorbereiteten Prüflingen werden Abschnitte von 5 × 5 cm einzeln in Petrischalen auf einen Traubenzucker-Nährboden aufgelegt und mit einer Sporensuspension der nachstehenden Pilze kontaminiert: *Aspergillus niger, Aureobasidium pullulans, Alternaria speciales, Penicillium citrinum, Strachybotrys atra, Paecilomyces varioti, Cladosporium herbarum, Aspergillus ustus* und *Aspergillus flavus.*

Die kontaminierten Schalen werden bei 28-30° C und 90-95% rel. Luftfeuchtigkeit gelagert, nach 3 Wochen wird ausgewertet. Anstriche gelten als schimmelfest, wenn die Prüflinge nach diesen Tests pilzfrei bleiben.

Nach der oben angegebenen Testmethode wird eine handelsübliche, alkalisch reagierende Dispersionsfarbe auf Polyvinylacetatbasis auf Schimmelfestigkeit geprüft.

Proben des Anstrichmittels, die, bezogen auf Gesamtfeststoffgehalt, 1,5 bis 2% (oder mehr) 3-(3-Jodpropargyl)-benzo-1,2,3-triazin-4-on enthalten, liefern sehr gut schimmelfeste Anstriche; auch dann, wenn die Anstriche zuvor den obengenannten Belastungen unterzogen wurden.

## Patentansprüche

1. 3-(3-Jodpropargyl)-benzo-1,2,3-triazin-4-one der Formel:

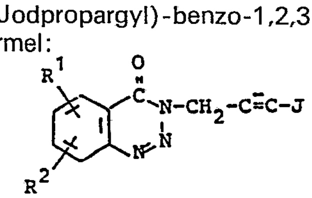

in der

$R^1$ Wasserstoff, $C_1$-$C_6$-Niederalkyl, Halogen, Cyano, Nitro, $C_1$-$C_6$-Niederalkoxy, $C_6$-$C_{18}$-Aryloxy, $C_1$-$C_6$-Niederalkylthio, $C_6$-$C_{18}$-Arylthio, Chlorphenylthio, $C_1$-$C_{10}$-Alkoxycarbonyl, $C_1$-$C_6$-Niederalkylsulfonyl, $C_6$-$C_{18}$-Arylsulfonyl oder Chlorphenylsulfonyl bedeutet, und

$R^2$ Wasserstoff, $C_1$-$C_6$-Niederalkyl oder Halogen bedeutet.

2. 3-(3-Jodpropargyl)-benzo-1,2,3-triazin-4-one nach Anspruch 1 der Formel:

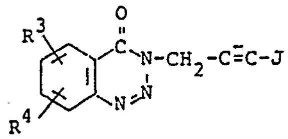

in der

$R^3$ Wasserstoff, $C_1$-$C_6$-Niederalkyl, Fluor, Chlor, Brom oder Nitro bedeutet, und

$R^4$ Wasserstoff, $C_1$-$C_6$-Niederalkyl oder Chlor bedeutet.

3. Verfahren zur Herstellung von 3-(3-Jodpropargyl)-benzo-1,2,3-triazin-4-onen gemäss Anspruch 1, dadurch gekennzeichnet, dass man Benzo-1,2,3-triazin-4-one der Formel:

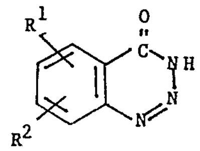

in der $R^1$ und $R^2$ die in Anspruch gegebenen Bedeutungen haben

mit einer Propargylverbindung der Formel:

$$X^1-CH_2-C\equiv C-J$$

in der $X^1$ Halogen, Alkylsulfonyloxy oder Arylsulfonyloxy bedeutet,

in Gegenwart einer Base umsetzt.

4. Verfahren zur Herstellung von 3-(3-Jodpropargyl)-benzo-1,2,3-triazin-4-onen gemäss Anspruch 1, dadurch gekennzeichnet, dass man Salze der Benzo-1,2,3-triazin-4-one der Formel:

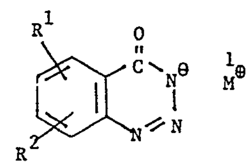

in der

$R^1$ und $R^2$ die in Anspruch 1 gegebenen Bedeutungen haben, und

$M^{1\oplus}$ für ein Metallion steht,

mit einer Propargylverbindung der Formel:

$$X^1-CH_2-C\equiv C-J$$

in der $X^1$ für Halogen, Alkylsulfonyloxy oder Arylsulfonyloxy steht,

umsetzt.

5. Verfahren nach den Ansprüchen 3 und 4, dadurch gekennzeichnet, dass es im Temperaturbereich von 20 bis 100° C durchgeführt wird.

6. Mikrobizides Mittel, enthaltend 3-(3-Jod-

propargyl)-benzo-1,2,3-triazin-4-one der Formel:

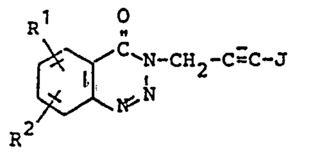

in der $R^1$ und $R^2$ die in Anspruch 1 gegebenen Bedeutungen haben.

7. Mikrobizides Mittel nach Anspruch 5, enthaltend ein 3-(3-Jodpropargyl)-benzo-1,2,3-triazin-4-on in einer Menge von 5 bis 100 Gew.-%.

8. Verwendung von mikrobiziden Mitteln nach Anspruch 5, zum Schutz technischer Materialien.

9. Verwendung von mikrobiziden Mitteln nach Anspruch 5 in einer Menge von 0,001 bis 5 Gew.-%, bezogen auf das zu schützende Material.

## Claims

1. 3-(3-Iodopropargyl)benzo-1,2,3-triazin-4-ones of the formula

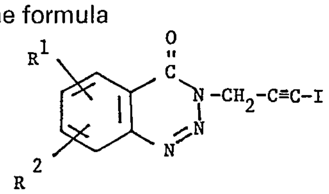

in which

$R^1$ denotes hydrogen, $C_1$-$C_6$-lower alkyl, halogen, cyano, nitro, $C_1$-$C_6$-lower alkoxy, $C_6$-$C_{18}$-aryloxy, $C_1$-$C_6$-lower alkylthio, $C_6$-$C_{18}$-arylthio, chlorophenylthio, $C_1$- $C_{10}$-alkoxycarbonyl, $C_1$-$C_6$-lower alkylsulphonyl, $C_6$-$C_{18}$-arylsulphonyl or chlorophenylsulphonyl, and

$R^2$ denotes hydrogen, $C_1$-$C_6$-lower alkyl or halogen.

2. 3-(3-Iodopropargyl)benzo-1,2,3-triazin-4-ones according to Claim 1 of the formula

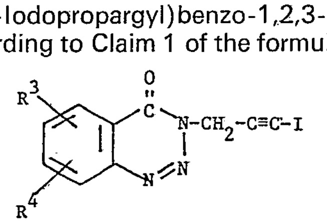

in which

$R^3$ denotes hydrogen, $C_1$-$C_6$-lower alkyl, fluorine, chlorine, bromine or nitro, and

$R^4$ denotes hydrogen, $C_1$-$C_6$-lower alkyl or chlorine.

3. Process for the preparation of 3-(3-iodopropargyl)benzo-1,2,3-triazin-4-ones according to Claim 1, characterised in that benzo-1,2,3-triazin-4-ones of the formula

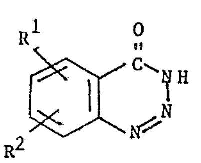

in which $R^1$ and $R^2$ have the meanings given in claim,

are reacted with a propargyl compound of the formula

$$X^1-CH_2-C\equiv C-I$$

in which $X^1$ denotes halogen, alkylsulphonyloxy or arylsulphonyloxy,

in the presence of a base.

4. Process for the preparation of 3-(3-iodopropargyl)benzo-1,2,3-triazin-4-ones according to Claim 1, characterised in that salts of benzo-1,2,3-triazin-4-ones of the formula

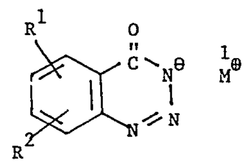

in which

$R^1$ and $R^2$ have the meanings given in Claim 1, and

$M^{1\oplus}$ represents a metal ion,

are reacted with a propargyl compound of the formula

$$X^1-CH_2-C\equiv C-I$$

in which $X^1$ represents halogen, alkylsulphonyloxy or arylsulphonyloxy.

5. Process according to Claims 3 and 4, characterised in that it is carried out in the temperature range of 20 to 100° C.

6. Microbicidal agent, containing 3-(3-iodopropargyl)benzo-1,2,3-triazin-4-ones of the for mula

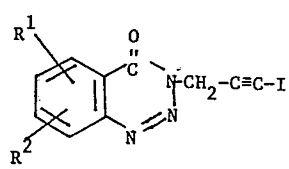

in which $R^1$ and $R^2$ have the meanings given in Claim 1.

7. Microbicidal agent according to Claim 5, containing a 3-(3-iodopropargyl)benzo-1,2,3-triazin-4-one in a quantity of 5 to 100% by weight.

8. Use of microbicidal agents according to Claim 5 for protecting industrial materials.

9. Use of microbicidal agents according to Claim 5 in a quantity of 0.001 to 5% by weight, based on the material to be protected.

## Revendications

1. 3-(3-Iodopropargyl)benzo-1,2,3-triazine-4-ones de formule

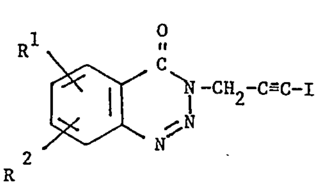

dans laquelle

$R^1$ représente l'hydrogène, un groupe alkyle inférieur en $C_1$-$C_6$, un halogène,un groupe cyano, nitro, alcoxy inférieur en $C_1$-$C_6$, aryloxy en $C_6$-$C_{18}$, alkylthio inférieur en $C_1$-$C_6$, arylthio en $C_6$-$C_{18}$, chlorophénylthio, (alcoxy en $C_1$-$C_{10}$)-

carbonyle, alkylsulfonyle inférieur en $C_1$-$C_6$, arylsulfonyle en $C_6$-$C_{15}$ ou chlorophénylsulfonyle, et

$R^2$ représente l'hydrogène, un groupe alkyle inférieur en $C_1$-$C_6$ ou un halogène.

2. 3-(3-Iodopropargyl)benzo-1,2,3-triazine-4-ones selon la revendication 1, de formule

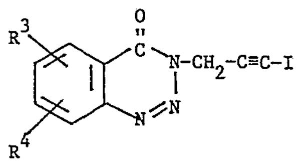

dans laquelle

$R^3$ représente l'hydrogène, un groupe alkyle inférieur en $C_1$-$C_6$, le fluor, le chlore, le brome ou un groupe nitro, et

$R^4$ représente l'hydrogène, un groupe alkyle inférieur en $C_1$-$C_6$ ou le chlore.

3. Procédé de préparation des 3-(3-iodopropargyl)benzo-1,2,3-triazine-4-ones selon la revendication 1, caractérisé en ce que l'on fait réagir en présence d'une base des benzo-1,2,3-triazine-4-ones de formule

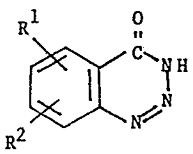

dans laquelle $R^1$ et $R^2$ ont les significations indiquées dans la revendication

avec un dérivé propargylique de formule

$$X^1-CH_2-C\equiv C-I$$

dans laquelle $X^1$ représente un halogène, un groupe alkylsulfonyloxy ou arylsulfonyloxy.

4. Procédé de préparation des 3-(3-iodopropargyl)benzo-1,2,3-triazine-4-ones selon la revendication 1, caractérisé en ce que l'on fait réagir des sels des benzo-1,2,3-triazine-4-ones de formule

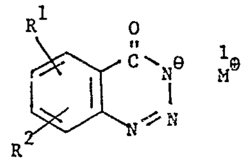

dans laquelle

$R^1$ et $R^2$ ont les significations indiquées dans la revendication 1, et

$M^{1\oplus}$ représente un ion métallique,

avec un dérivé propargylique de formule

$$X^1-CH_2-C\equiv C-I$$

dans laquelle $X^1$ représente un halogène, un groupe alkylsulfonyloxy ou arylsulfonyloxy.

5. Procédé selon les revendications 3 et 4, caractérisé en ce que l'on opère dans l'intervalle de températures de 20 à 100° C.

6. Produit microbicide contenant des 3-(3-iodopropargyl)benzo-1,2,3-triazine-4-ones de formule

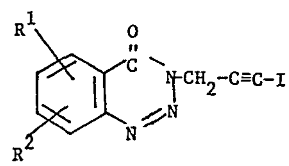

dans laquelle $R^1$ et $R^2$ ont les significations indiquées dans la revendication 1.

7. Produit microbicide selon la revendication 5, contenant une 3-(3-iodopropargyl)benzo-1,2,3-triazine-4-one en quantité de 5 à 100% en poids.

8. Utilisation des produits microbicides selon la revendication 5 pour la protection des matériaux techniques.

9. Utilisation des produits microbicides selon la revendication 5 en quantité de 0,001 à 5% en poids par rapport au matériau à protéger.